# EUROPEAN PATENT SPECIFICATION

(11) **EP 4 321 192 B1**
(45) Date of publication and mention of the grant of the patent: **07.01.2026**
(21) Application number: 22461592.2
(22) Date of filing: 11.08.2022
(51) Int. Cl.: A61M 15/00

(54) **A DEVICE FOR MONITORING USE OF AN INHALER**
VORRICHTUNG ZUR ÜBERWACHUNG DER VERWENDUNG EINES INHALATORS
DISPOSITIF DE SURVEILLANCE DE L'UTILISATION D'UN INHALATEUR

(43) Date of publication of application: 14.02.2024
(73) Proprietor: Findair Sp. z o. o., 31-235 Krakow (PL)
(72) Inventor: MIKOSZ, Jacek, 31-162 Krakow (PL); CZYZ, Michal, 30-719 Krakow (PL); MIKOSZ, Tomasz, 32-091 Michalowice (PL)
(74) Representative: Kancelaria Eupatent.pl Sp. z o.o.

(56) References cited:
- WO-A1-2019/012558
- US-A1- 2007 251 950
- US-A1- 2007 295 329
- US-A1- 2017 100 551
- US-A1- 2017 325 734
- US-A1- 2019 385 727
- US-A1- 2021 085 564

## Description

### TECHNICAL FIELD

The present invention relates to a device for monitoring use of an inhaler.

### BACKGROUND

An inhaler (also known as a puffer, pump, allergy spray or powder dispenser) is a medical device used for delivering medicines into the lungs through the work of a person's breathing. This allows medicines to be delivered to and absorbed in the lungs, which provides the ability for targeted medical treatment to this specific region of the body, as well as a reduction in the side effects of oral medications. Some of the common types of inhalers include metered-dose inhalers, dry powder inhalers, soft mist inhalers, and nebulizers.

For example, one type of inhaler is a pressurized metered-dose inhaler (MDI or pMDI) which comprises three main components - a metal canister, a plastic actuator, and a metering valve. The medication is typically stored in solution in the pressurized canister that contains a propellant or a suspension. The MDI canister is attached to the plastic, hand-operated actuator. On activation, the metered-dose inhaler releases a fixed dose of medication in aerosol form through the actuator and into a patient's lungs. These devices require significant coordination as a person must discharge the medication at or near the same time that they inhale it in order for the medication to be effective.

Most inhalers are not washable or sterilizable. That is why inhalers are designed to have a limited lifetime. Manufacturers of current pMDI aim for low costs. Manufacturing inhalers with additional components such as electronic or mechanical counters or radio transmitters is expensive and currently not very cost-effective.

Depending on the stage of illness, duration of use, or therapy, using an inhaler presents different challenges which change over time. In the early stages, the key problem is learning the inhalation technique and how to coordinate holding the inhaler with the right phase of inhalation. In the later stages, the key element is adherence to the assigned therapy as prescribed. There is also an increasingly important role for information on drug use in the clinical context. Such data can be used to assess disease progression and the effectiveness of prescribed therapy. Due to increasing technological capabilities, there is an emerging opportunity to measure data in an automated manner that would have previously had to be recorded manually.

There are known inhalers for which dedicated monitoring devices have been developed. These monitoring devices are adapted to be attachable to a particular type of inhaler. Therefore, after the medicine is used and a user buys a new inhaler, the monitoring device can be attached to the new inhaler.

However, dedicated monitoring devices tend to be expensive. Moreover, especially in early stage of treatment, when a user may be required to test various types of inhalers to determine the one that is most optimal for treatment of user's condition, purchasing a dedicated monitoring device each time a new type of inhaler is used can be very costly.

WO2019012558A1 discloses an adherence-monitoring and tracking device for capturing usage of a metered dose inhaler (MDI). The device comprises an enclosure for enclosing a metered dose inhaler, and an electro-mechanical system forming a part of enclosure to sense and log the operation of the MDI. The electro-mechanical system comprises at-least one transducer to capture one or more parameters pertaining to an operation of the MDI by the user. A microcontroller processes said captured parameters, and an output-unit renders data pertaining to operation of the MDI.

### SUMMARY OF THE INVENTION

There is a need to provide a device for monitoring use of an inhaler, which could be used with different types of inhalers.

The invention relates to a device for monitoring use of an inhaler, according to the appended claims.

The device according to the invention can be used with replaceable holder modules to couple it with various kinds of inhalers. The pivotable arm with a sensor can be mounted on an inhaler such as to detect its state of use or non-use and therefore provide measurement data to operating modules within the main module. The replaceable holder modules are simple mechanical structures which can be provided at little cost, while the main costly components are mounted within the main module.

These and other features, aspects and advantages of the invention will become better understood with reference to the following drawings, descriptions and claims.

### BRIEF DESCRIPTION OF DRAWINGS

The present invention is shown by means of preferable embodiments in a drawing, wherein:
Fig. 1 presents an embodiment of a main module of the monitoring device;
Fig. 2 presents examples of operating modules;
Figs. 3A-3C present the monitoring device with a first embodiment of a holder module;
Figs. 4A-4C present the monitoring device with a second embodiment of the holder module;
Figs. 5A-5C present the monitoring device with a third embodiment of the holder module;
Figs. 6A-6C present the monitoring device with a fourth embodiment of the holder module.

### DETAILED DESCRIPTION

The following detailed description is of the best currently contemplated modes of carrying out the invention. The description is not to be taken in a limiting sense but is made merely for the purpose of illustrating the general principles of the invention.

The monitoring device comprises a main module 10 and a holder module 30-60 attachable to the main module 10.

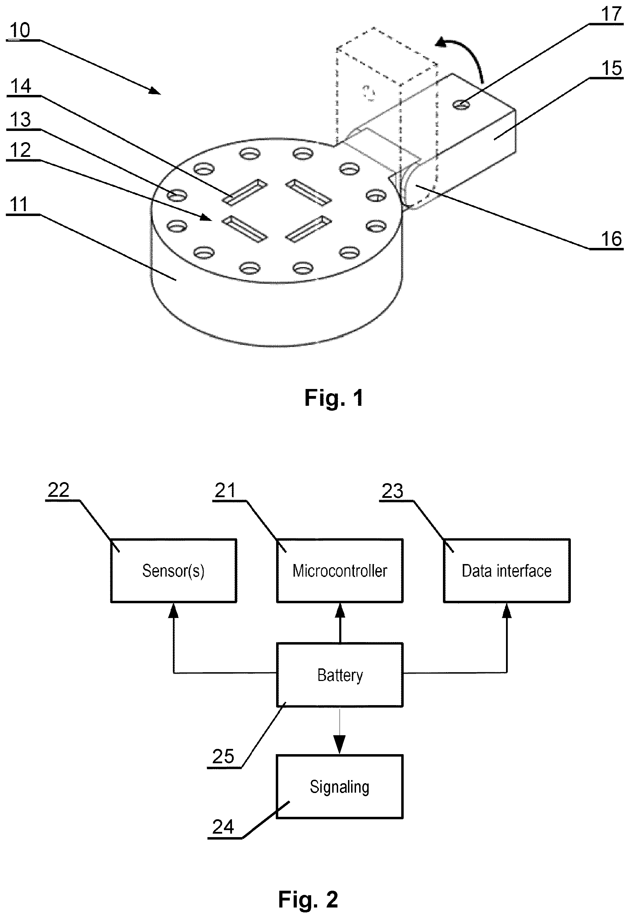

The main module 10, as shown in Fig. 1, has a body 11 for accommodating operating modules 21-24 therein, as will be explained further below. The body 11 preferably has a flat cylindrical shape, as this is optimal for accommodating necessary modules and can be easily attached to most types of inhalers, though other shapes of the body are possible, such as cubical, ball-shaped etc.

The body 11 has attachment means 12 for attaching a holder module 30-60 to the body 11. For example, the main module attachment means 12 may have a form of a plurality of cylindrical holes 13 and a plurality of slots 14, wherein the holes 13 are arranged along a circumference of a top flat surface of the cylindrical body 11 and the slots 14 are arranged in the middle of that surface, arranged in perpendicular to each other, as shown in the embodiment of Fig. 1. However, other types of attachment means 12 are possible as well, such as a plurality of protrusions, or magnets or hooks. The holder module 30-60, as will be explained further below, comprises holder module attachment means 32-62 compatible with the main module attachment means 12, so that the modules can be coupled by a form fit connection, magnetic connection, snap fit connection etc. or a combination of different types of connections.

The body 11 further comprises a pivotable arm 15 which can pivot along a hinge 16, for example by 90 degrees (as shown in Fig. 1) or another angle such as 180 degrees. The pivotable arm 15 comprises a proximity sensor 17 mounted therein.

The sensor 17 can be of a variety of types. For example, the sensor 17 can be a microphone, a pressure sensor, a pressing force sensor (such as a mechanic or electronic button, such as a press-button or a switch), magnetic field sensor (such as a Hall sensor), a light sensor, a temperature sensor, an image registering module, a humidity sensor, an accelerometer, a gyroscope, a magnetometer, a volatile particles (VOC) sensor, an alcohol sensor, a capacitive sensor (such as a touch sensor), a proximity ultrasound or infrared sensor, a resistive pressing force sensor, a tensometer, a piezoelectric sensor, or a set of two or more of the aforementioned sensors.

At least one of the sensors 17, 22 is configured to detect use and/or non-use of the inhaler.

According to the invention, the sensor 17 is a proximity sensor 17 configured to detect whether there is an object proximal thereto, i.e., within a specific detection distance, such as 1 mm, 5 mm or 10 mm. The detection distance of the proximity sensor 17 can be adjustable.

The operating modules accommodated within the main module 10 include, as shown in Fig. 2, a microcontroller 21 to which at least one sensor 17, 22 is connected. The microcontroller 21 is configured to receive measurements made by the at least one sensor 17, 22, optionally process these measurements (for example, by collecting measurements over a specific time period, pre-processing measurements to filter out noise etc.) and provide an output to a data interface 23. The data interface 23 can be a wireless communication module using a common standard to connect to a user device, such as a computer, a smartphone, or to a cloud server. For example, the wireless data interface 23 can operate according to one of known standards such as WiFi, Bluetooth etc. Alternatively, a wired data interface 23 can be used. The data interface 23 can be a separate module or a module integrated into the microcontroller 21. A signaling module 24, such as a sound generator, a buzzer, a light (such as a LED diode), a display is configured to provide information to the user. The modules 21-24 are powered from a battery 25. The battery 25 can be replaceable or can be charged via a dedicated charging port.

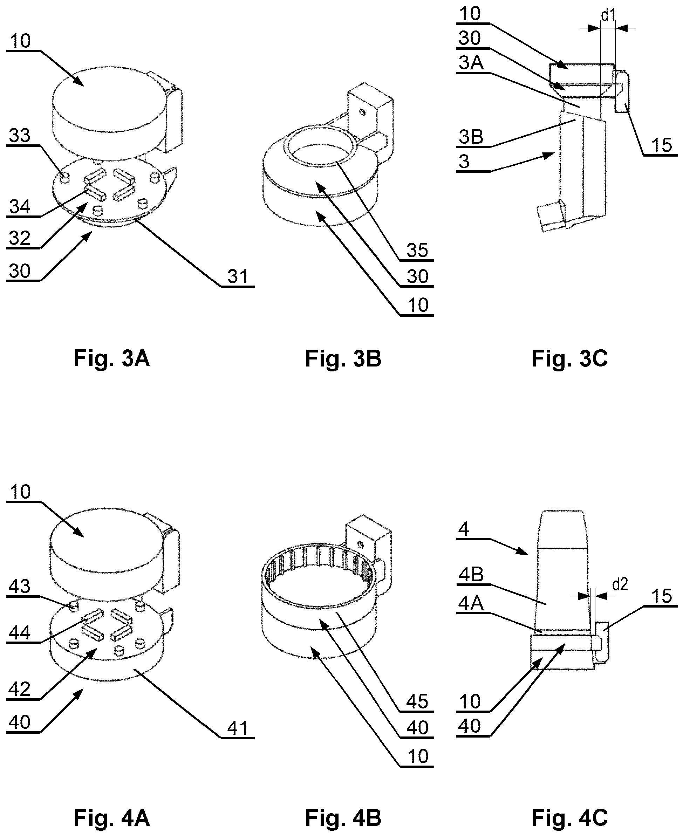

Figs. 3A-3C present the monitoring device with a first embodiment of the holder module 30, wherein Fig. 3A shows the modules 10, 30 before assembly, fig. 3B shows the modules 10, 30 assembled to form the device and Fig. 3C shows the device coupled to an inhaler. The holder module 30 has a body 31 comprising holder module attachment means 32 in a form of protrusions 33, 34 compatible with the holes 13 and slots 14 of the main module attachment means 12. The body 31 has formed therein an inhaler coupler 35 in a form of a protruded ring of an internal diameter corresponding to an external diameter of a canister 3A of a pMDI inhaler 3. Thereby, the device can be coupled to the pMDI inhaler 3 by mounting it on the canister 3A, with the pivotable arm 15 set to a position such that the proximity sensor 17 can detect two states: a first "non-use" state, wherein the proximity sensor 17 faces the canister 3A (as shown in Fig. 3C), and a second "use" state wherein the canister 3A is pressed downwards within the body 3B of the inhaler, such that the proximity sensor 17 faces the inhaler body 3B, which can be detected as a closer distance by the proximity sensor 17. In such a case, the proximity sensor 17 can have its detection distance set to a value shorter than a distance d1 between the proximity sensor 17 and the canister 3A, so that when the canister 3A is pressed, the proximity sensor 17 can detect the presence of the inhaler body 3B which will appear within the detection distance d1. Therefore, use of the inhaler is detected by detecting presence of inhaler body 3B within the detection distance d1.

Figs. 4A-4C present the monitoring device with a second embodiment of the holder module 40. The holder module 40 has a body 41 comprising holder module attachment means 42 in a form of protrusions 43, 44 compatible with the holes 13 and slots 14 of the main module attachment means 12. The body 41 has formed therein an inhaler coupler 45 in a form of a protruded ring having internal diameter equal to an external diameter of a cap 4A of a Turbohaler inhaler 4. Thereby, the device can be coupled to the Turbohaler inhaler 4 by mounting it on the cap 4A, with the pivotable arm 15 set to a position such that the proximity sensor 17 can detect two states: a first "non-use" state, wherein the proximity sensor 17 faces the body 4B (as shown in Fig. 4C) of the inhaler, and a second "use" state wherein the cap 4A is removed and the proximity sensor 17 does not detect the proximity of the inhaler body 4B. In such a case, the proximity sensor 17 can have its detection distance set to a value approximate to a distance d2 between the proximity sensor 17 and the inhaler body 4B, so that when the device is removed from the inhaler 4 along with the cap 4A, the proximity sensor 17 can stop detecting the presence of the inhaler body 4B which no longer appears within the detection distance d2. Therefore, use of the inhaler is detected by detecting non-presence of inhaler body 4B within the detection distance d2.

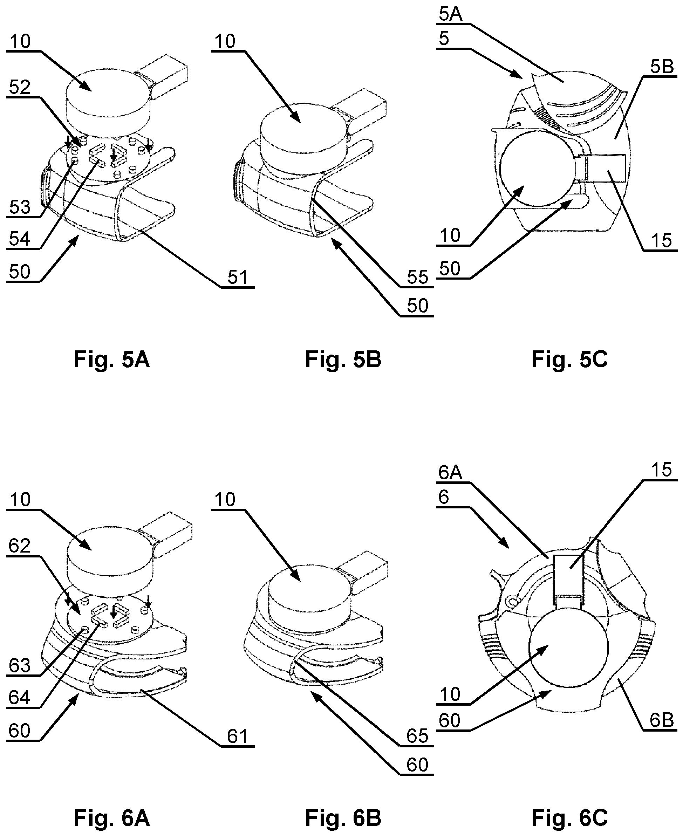

Figs. 5A-5C present the monitoring device with a third embodiment of the holder module 50. The holder module 50 has a body 51 comprising holder module attachment means 52 in a form of protrusions 53, 54 compatible with the holes 13 and slots 14 of the main module attachment means 12. The body 51 has formed therein an inhaler coupler 55 in a form of a clamp with elastic arms configured to attach the holder module 50 to a body of a Ellipta inhaler 5. Thereby, the device can be coupled to the Ellipta inhaler 5 by mounting it on the inhaler body 5B, with the pivotable arm 15 set to a position such that the proximity sensor 17 is present in an area to which a pivotable cap 5A of the inhaler 5 is moved during use of the inhaler. Therefore, the proximity sensor 17 can detect two states: a first "non-use" state, wherein the proximity sensor 17 faces the body 5B (as shown in Fig. 5C) of the inhaler, and a second "use" state wherein the cap 5A is folded away towards the area within the line of detection of the proximity sensor 17. In such a case, the proximity sensor 17 can have its detection distance set to a value closer than a distance between the proximity sensor 17 and the inhaler body 5B, so that when the cap 5A is folded away, the proximity sensor can detect its presence. Therefore, use of the inhaler is detected by detecting presence of the cap 5A within the detection distance.

Figs. 6A-6C present the monitoring device with a third embodiment of the holder module 60. The holder module 60 has a body 61 comprising holder module attachment means 62 in a form of protrusions 63, 64 compatible with the holes 13 and slots 14 of the main module attachment means 12. The body 61 has formed therein an inhaler coupler 65 in a form of a clamp with elastic arms configured to attach the holder module 60 to a body of a Diskus inhaler 6. Thereby, the device can be coupled to the Diskus inhaler 6 by mounting it on the inhaler body 6B, with the pivotable arm 15 set to a position such that the proximity sensor 17 is present in an area within which a rotatable portion 6A of the inhaler 6 operates. Therefore, the proximity sensor 17 can detect two states: a first "non-use" state, wherein the proximity sensor 17 faces one fragment of the rotatable portion 6A (as shown in Fig. 6C) of the inhaler, and a second "use" state wherein the rotatable portion 6A is rotated to a position wherein it has a different shape and the proximity sensor 17 can detect a change of distance that it perceives.

As shown in Figs. 3A-3C to 6A-6C, all the holder module attachment means 32, 42, 52, 62 are compatible with the main module attachment means 12 in so far as they can be coupled together, but the configuration of protrusions 33-34 to 63-64 is different. Thereby, the main module attachment means 12 may comprise sensors for detecting which portion of the main module attachment means 12 is coupled with the holder module attachment means 62, thereby detecting the type of the holder module 30-60 to which the main module 10 is attached, and thereby the type of inhaler to be monitored. This information can be next used to configure the microcontroller 21 operation, for example to determine which type of sensors 22 shall be used to monitor the inhaler and to configure the detection distance of the proximity sensor 17.

The microcontroller 21 allows to monitor the inhalation process based on data from measurement sensors 17, 22. In a simple embodiment, the device may be configured to simply measure when the device was used by means of one of the sensors 17, 22, such as by means of detecting change of state of the proximity sensor 17. For example, it can register a date and time of use (in particular, the start and end of use). However, if more sensors 22 are installed, various other parameters can be measured, depending on the type of sensor 22 used. For example, the sensors 22 may include a microphone, a pressure sensor, a light sensor, a temperature sensor, a camera, a gas sensor, a volatile components (VOC) sensor, an alcohol sensor, a magnetic force sensor such as a Hall sensor, a humidity sensor, an accelerometer, a gyroscope, a magnetometer, a capacitive sensor such as a touch sensor, an ultrasound proximity sensor, an infrared proximity sensor, a resistive pressure sensor, a tensometer, a piezoelectric sensor.

Moreover, the main module may comprise further components, such as a video recorder, an NFC module, an RFID module, a press sensor such as a mechanical and/or electric button or a switch.

While the invention has been described with respect to a limited number of embodiments, it will be appreciated that many variations, modifications and other applications of the invention may be made. Therefore, the claimed invention as recited in the claims that follow is not limited to the embodiments described herein.

## Claims

1. A device for monitoring use of an inhaler (3, 4, 5, 6), the device comprising:
- a main module (10) having a body (11) accommodating operating modules (21-24), the body (11) comprising main module attachment means (12) and a pivotable arm (15) with a sensor (17);
- a microcontroller (21) accommodated in the main module (10) and configured to receive measurements made by the sensor (17) to determine when the device was used; and
- a holder module (30, 40, 50, 60) having a body (31, 41, 51, 61) comprising holder module attachment means (32, 42, 52, 62) compatible with the main module attachment means (12) and an inhaler coupler (35, 45, 55, 65) configured to couple the holder module (30, 40, 50, 60) to the inhaler (3, 4, 5, 6);
**characterized in that**
- the sensor (17) is a proximity sensor for detecting a distance between an element of the inhaler (3, 4, 5, 6) and the proximity sensor (17);
- the microcontroller (21) is configured to determine when the device was used based on the information on the detected distance received from the sensor (17); and
- the device is configured to detect state of use or non-use of the inhaler (3,4,5,6) based on the detected presence or non-presence of the element of the inhaler (3,4,5,6) within a detection distance (d1).

2. The device according to claim 1, wherein the operating modules (21-24) are powered from a battery (25) and the microcontroller (21) is configured to transfer the measurement data to a data interface (23).

3. The device according to any of previous claims, wherein the main module attachment means (12), and the holder module attachment means (32, 42, 52, 62) are configured to be coupled together by form fit connection.

4. The device according to any of previous claims, wherein the main module attachment means (12), and the holder module attachment means (32, 42, 52, 62) are configured to be coupled together by magnetic connection.

5. The device according to any of previous claims, wherein the main module attachment means (12), and the holder module attachment means (32, 42, 52, 62) are configured to be coupled together by snap fit connection.

6. The device according to any of previous claims, wherein the proximity sensor (17) has an adjustable detection distance.

7. The device according to any of previous claims, wherein the main module attachment means (12) comprise sensors for detecting which portion of the main module attachment means (12) is coupled with the holder module attachment means (32, 42, 52, 62).

8. The device according to any of previous claims, wherein the main module (10) further comprises a signaling module (25).

## Patentansprüche

1. Vorrichtung zur Überwachung der Verwendung eines Inhalators (3, 4, 5, 6), wobei die Vorrichtung Folgendes umfasst:
- ein Hauptmodul (10), das einen Körper (11) aufweist, der Betriebsmodule (21-24) aufnimmt, wobei der Körper (11) Hauptmodulanbringungsmittel (12) und einen schwenkbaren Arm (15) mit einem Sensor (17) umfasst;
- einen Mikrocontroller (21), der in dem Hauptmodul (10) aufgenommen und dazu konfiguriert ist, von dem Sensor (17) durchgeführte Messungen zu empfangen, um zu bestimmen, wann die Vorrichtung verwendet wurde; und
- ein Haltermodul (30, 40, 50, 60), das einen Körper (31, 41, 51, 61) aufweist, der Haltermodulanbringungsmittel (32, 42, 52, 62), die mit den Hauptmodulanbringungsmitteln (12) kompatibel sind, und einen Inhalatorkoppler (35, 45, 55, 65) umfasst, der dazu konfiguriert ist, das Haltermodul (30, 40, 50, 60) an den Inhalator (3, 4, 5, 6) zu koppeln;
**dadurch gekennzeichnet, dass**
- der Sensor (17) ein Näherungssensor zum Detektieren einer Entfernung zwischen einem Element des Inhalators (3, 4, 5, 6) und dem Näherungssensor (17) ist;
- der Mikrocontroller (21) dazu konfiguriert ist, auf Grundlage der von dem Sensor (17) empfangenen Informationen über die detektierte Entfernung zu bestimmen, wann die Vorrichtung verwendet wurde; und
- die Vorrichtung dazu konfiguriert ist, einen Zustand der Verwendung oder Nichtverwendung des Inhalators (3,4,5,6) auf Grundlage der detektierten Anwesenheit oder Nichtanwesenheit des Elements des Inhalators (3,4,5,6) innerhalb einer Detektionsentfernung (d1) zu detektieren.

2. Vorrichtung nach Anspruch 1, wobei die Betriebsmodule (21-24) von einer Batterie (25) mit Leistung versorgt werden und der Mikrocontroller (21) dazu konfiguriert ist, die Messdaten an eine Datenschnittstelle (23) zu übertragen.

3. Vorrichtung nach einem der vorhergehenden Ansprüche, wobei die Hauptmodulanbringungsmittel (12) und die Haltermodulanbringungsmittel (32, 42, 52, 62) dazu konfiguriert sind, durch eine Formschlussverbindung miteinander gekoppelt zu werden.

4. Vorrichtung nach einem der vorhergehenden Ansprüche, wobei die Hauptmodulanbringungsmittel (12) und die Haltermodulanbringungsmittel (32, 42, 52, 62) dazu konfiguriert sind, durch eine magnetische Verbindung miteinander gekoppelt zu werden.

5. Vorrichtung nach einem der vorhergehenden Ansprüche, wobei die Hauptmodulanbringungsmittel (12) und die Haltermodulanbringungsmittel (32, 42, 52, 62) dazu konfiguriert sind, durch eine Schnappverbindung miteinander gekoppelt zu werden.

6. Verfahren nach einem der vorhergehenden Ansprüche, wobei der Näherungssensor (17) eine einstellbaren Detektionsentfernung aufweist.

7. Vorrichtung nach einem der vorhergehenden Ansprüche, wobei die Hauptmodulanbringungsmittel (12) Sensoren zum Detektieren umfassen, welcher Abschnitt der Hauptmodulanbringungsmittel (12) mit den Haltermodulanbringungsmitteln (32, 42, 52, 62) gekoppelt ist.

8. Verfahren nach einem der vorhergehenden Ansprüche,
wobei das Hauptmodul (10) ferner ein Signalisierungsmodul (25) umfasst.

## Revendications

1. Dispositif de surveillance de l'utilisation d'un inhalateur (3, 4, 5, 6), le dispositif comprenant :
- un module principal (10) ayant un corps (11) recevant des modules de fonctionnement (21 à 24), le corps (11) comprenant des moyens de fixation de module principal (12) et un bras pivotant (15) avec un capteur (17) ;
- un microcontrôleur (21) logé dans le module principal (10) et configuré pour recevoir les mesures effectuées par le capteur (17) afin de déterminer quand le dispositif a été utilisé ; et
- un module de support (30, 40, 50, 60) ayant un corps (31, 41, 51, 61) comprenant des moyens de fixation de module de support (32, 42, 52, 62) compatibles avec les moyens de fixation de module principal (12) et un coupleur d'inhalateur (35, 45, 55, 65) configuré pour coupler le module de support (30, 40, 50, 60) à l'inhalateur (3, 4, 5, 6) ;
**caractérisé en ce que**
- le capteur (17) est un capteur de proximité pour détecter une distance entre un élément de l'inhalateur (3, 4, 5, 6) et le capteur de proximité (17) ;
- le microcontrôleur (21) est configuré pour déterminer quand le dispositif a été utilisé sur la base des informations concernant la distance détectée reçues du capteur (17) ; et
- le dispositif est configuré pour détecter l'état d'utilisation ou de non-utilisation de l'inhalateur (3, 4, 5, 6) sur la base de la présence ou de la non-présence détectée de l'élément de l'inhalateur (3, 4, 5, 6) à une distance de détection (d1).

2. Dispositif selon la revendication 1, dans lequel les modules de fonctionnement (21 à 24) sont alimentés à partir d'une batterie (25) et le microcontrôleur (21) est configuré pour transférer les données de mesure à une interface de données (23).

3. Dispositif selon l'une quelconque des revendications précédentes, dans lequel les moyens de fixation de module principal (12) et les moyens de fixation de module de support (32, 42, 52, 62) sont configurés pour être couplés ensemble par un raccordement par ajustement de forme.

4. Dispositif selon l'une quelconque des revendications précédentes, dans lequel les moyens de fixation de module principal (12) et les moyens de fixation de module de support (32, 42, 52, 62) sont configurés pour être couplés ensemble par un raccordement magnétique.

5. Dispositif selon l'une quelconque des revendications précédentes, dans lequel les moyens de fixation de module principal (12) et les moyens de fixation de module de support (32, 42, 52, 62) sont configurés pour être couplés ensemble par un raccordement par encliquetage.

6. Dispositif selon l'une quelconque des revendications précédentes, dans lequel le capteur de proximité (17) présente une distance de détection réglable.

7. Dispositif selon l'une quelconque des revendications précédentes, dans lequel les moyens de fixation de module principal (12) comprennent des capteurs pour détecter quelle partie des moyens de fixation de module principal (12) est couplée aux moyens de fixation de module de support (32, 42, 52, 62).

8. Dispositif selon l'une quelconque des revendications précédentes,
dans lequel le module principal (10) comprend en outre un module de signalisation (25).
